Europäisches Patentamt

European Patent Office

Office européen des brevets

(11) Publication number: **0 489 931 A1**

## EUROPEAN PATENT APPLICATION
### published in accordance with Art. 158(3) EPC

(21) Application number: **91912070.9**

(22) Date of filing: **28.06.91**

(86) International application number:
**PCT/JP91/00874**

(87) International publication number:
**WO 92/00089 (09.01.92 92/02)**

(51) Int. Cl.5: **A61K 37/02**, A61K 39/395,
A61K 45/00

(30) Priority: **29.06.90 JP 173516/90**

(43) Date of publication of application:
**17.06.92 Bulletin 92/25**

(84) Designated Contracting States:
**AT BE CH DE DK ES FR GB GR IT LI LU NL SE**

(71) Applicant: **TORAY INDUSTRIES, INC.**
**2-1, Nihonbashi Muromachi 2-chome**
**Chuo-ku**
**Tokyo 103(JP)**

(72) Inventor: **YAMAZAKI, Shojiro**
**B-2,2-24, Tsunishi 2-chome**
**Kamakura-shi, Kanagawa 248(JP)**
Inventor: **SONE, Saburo**
**1120-3, Yoshida-cho, Totsuka-ku**
**Yokohama-shi, Kanagawa 244(JP)**
Inventor: **KAJITA, Akemi**
**936-16, Daigiri**
**Fujisawa-shi, Kanagawa 251(JP)**

(74) Representative: **Kador & Partner**
**Corneliusstrasse 15**
**W-8000 München 5(DE)**

(54) **IMMUNOTOXIN COMPLEX.**

(57) An immunotoxin complex prepared by combining an A-chain part of toxin with an antibody which is readily combined with a tumor cell and incorporated therein. This complex combines specifically with a tumor cell to efficaciously kill the cell, thus being useful for treating cancer.

EP 0 489 931 A1

Fig. 1

This invention relates to an immunotoxin conjugate which is useful for treating cancers. More particularly, this invention relates to an immunotoxin conjugate having a monoclonal antibody which specifically binds to tumor cells, to which a cytotoxic component is bound.

BACKGROUND ART

Lung cancer is a malignant tumor of which incidence is extremely high in, for example, the U.S. According to the Report by U.S. Cancer Association, in 1989, 155,000 people newly caught lung cancer and it was estimated that 142,000 patients will die of lung cancer. The death rate among the patients suffering from lung cancer has been consistently increasing in these 30 years and lung cancer has become the cancer having the highest death rate in cancers. The lack of effective therapy contributes to the increase in the death rate of lung cancer.

Colon cancer has the second highest incidence next to lung cancer. In the U.S. in 1989, 151,000 people newly caught colon cancer and 61,300 patients were estimated to die of the cancer. The only hope to recover from this cancer is the surgical therapy at the initial stage of the cancer. However, once the cancer extends beyond the intestine wall, it is difficult to cure the cancer.

Since the forms of the above-mentioned cancers as well as hepatocarcinoma are extending, a novel composition for the therapy of major recurrent metastasizing diseases is demanded.

It is well-known to use a cytotoxic component for the therapy of cancers and the difficult problems associated with this therapy are also well-known. One of the largest problems in the difficult problems is that the cytotoxic component lacks specificity to cancers. To overcome this problem, various monoclonal antibodies which specifically bind to the tumor cells have been studied and it is now being tried to cure cancers by immunotoxin conjugates containing the monoclonal antibody and the cytotoxic component. However, the cytotoxicity of the cytotoxic component often affects not only to the tumor cells, but also to a part of the normal cells and normal tissues, especially liver and kidney.

The cytotoxic substances which are most widely employed are plant lectins such as ricin and abrin. A plant lectin consists of A chain and B chain which are cross-linked by disulfide bond. The A chain has an activity to inhibit the protein synthesis, that is, a cytotoxic activity in eukaryotic cells, and the B chain recognizes the sugar chains on the surface of the cells and binds to them. Once the lectin binds to the cell surface by the recognition by the B chain, the A chain is internalized into the cell to inhibit the protein synthesis, thereby finally necrotizes the cell.

With the conjugate consisting of the plant lectin ricin and a monoclonal antibody which specifically binds to the tumor cells, by the cell surface sugar chain-recognizing ability of the B chain, the conjugate is easily bound to the normal cells. Thus, the conjugate has a large drawback which causes side effects. Several countermeasures to this problem have been tried, which include a method in which a ricin having a B chain of which sugar-recognizing ability is blocked is used [e.g., S.E. Moroney, et al., Biochemistry, 26, 8390 (1987)], and a method in which a conjugate containing ricin A chain but not containing ricin B chain is employed [e.g., A. E. Frankel, ed., Immunotoxins, Kluwer Academic Publisher (1988)]. With the conjugate consisting of the ricin A chain alone bound to a monoclonal antibody specifically binding to the tumor cells, the ricin A chain is sometimes not internalized into the cells. In this case, although the adverse affect to the normal cells is also lowered, the cytotoxicity to the tumor cells is also reduced, so that the effectiveness of the conjugate is reduced. Thus, it is necessary to provide means for sending the ricin A chain into the cells.

The object of the present invention is to provide a substance by which the cytotoxic component is sent to the tumor cells and is internalized into the cells, thereby effectively killing the tumor cells, which is effective for the therapy of cancers.

DISCLOSURE OF THE INVENTION

The present invention provides a pharmaceutical for treating cancers used in chemotherapy of cancers, which is an immunotoxin conjugate prepared by chemically binding the A chain of a plant lectin such as ricin or abrin to a monoclonal antibody specific to tumor cells.

That is, the present invention provides an immunotoxin conjugate comprising an antibody which binds to a tumor cell and which has a property to be easily internalized into the tumor cell, and A chain of a toxin bound to the antibody.

BRIEF DESCRIPTION OF THE DRAWINGS

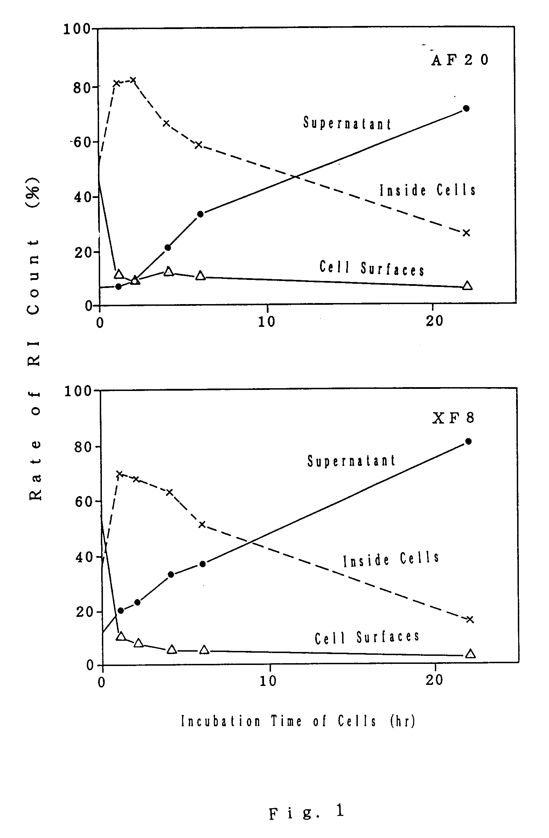

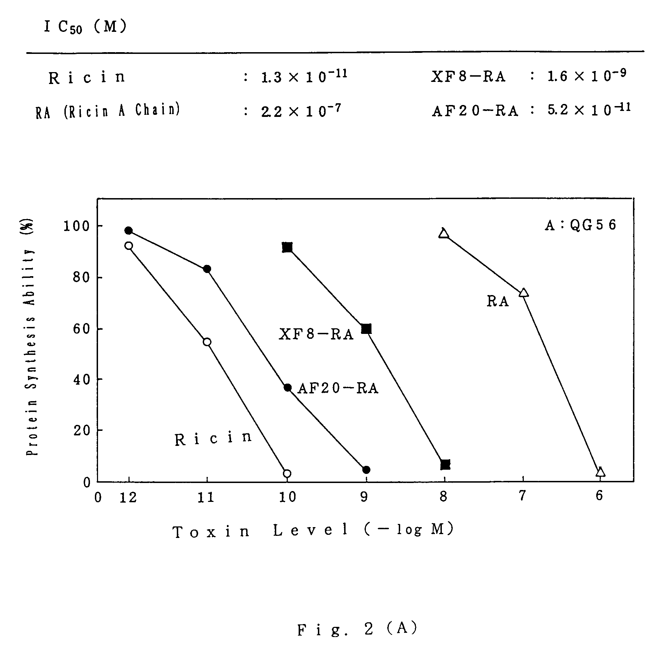

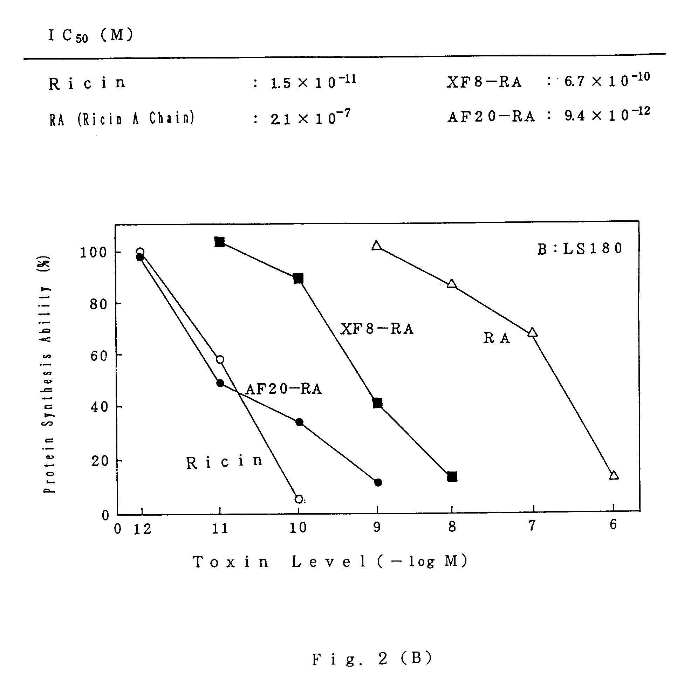

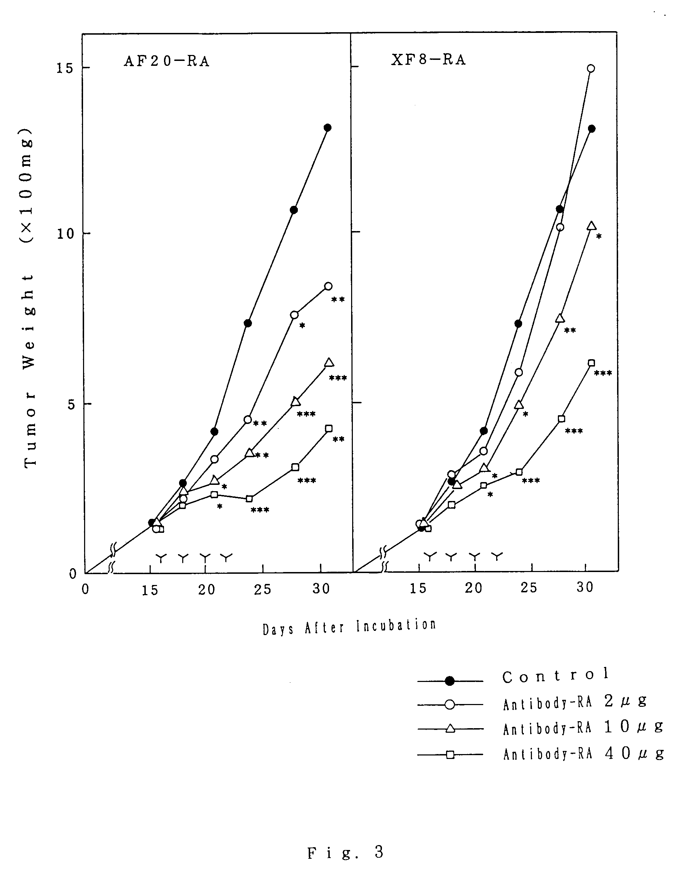

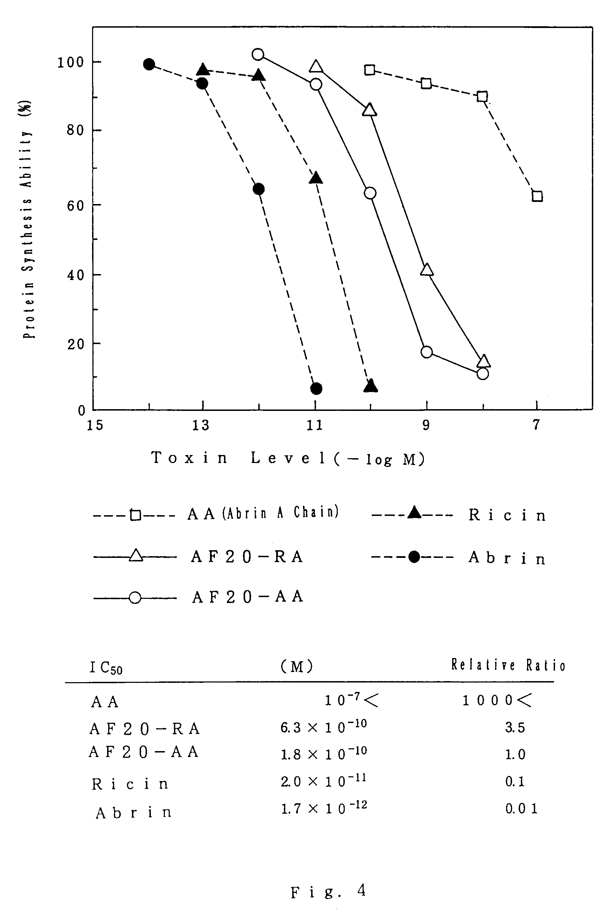

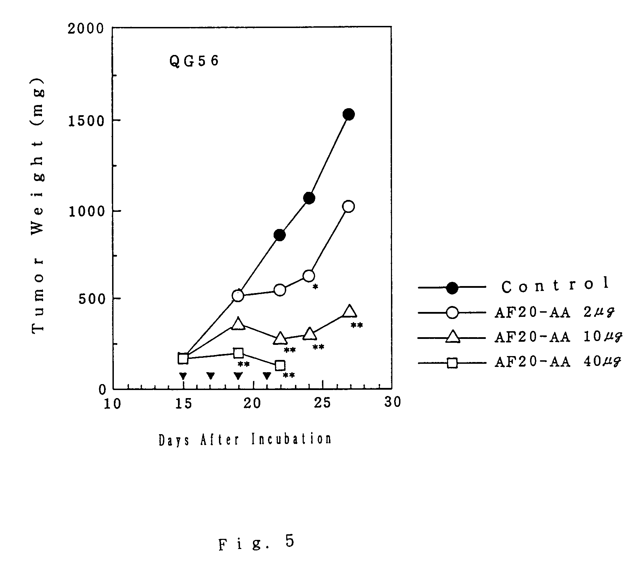

Fig. 1 shows the uptake of two antibodies (AF20 and XF8) into the carcinoma cells (QG-56) after the antibodies bound to the cells. Fig. 2 shows the results of the growth inhibitions of cancer cells by the two antibody-ricin A chain conjugates, wherein A shows the results about the human lung squamous carcinoma cells (QG-56) and B shows the results about the human colon adenocarcinoma cells (LS-180). Fig. 3 shows the changes in the weight of tumors (weight calculated from the diameters of the tumors) in nude mice (7 mice/group) bearing QG-56 carcinoma cells, which mice are treated with the two antibody-ricin A chain conjugates. Fig. 4 shows the growth inhibition of QG-56 cells by AF20-abrin A chain conjugate. Fig. 5 shows the change in the weight of tumor in nude mice bearing QG-56 carcinoma cells, to which AF20-abrin A chain conjugate was administered. In either of Figs. 3 and 5, the arrow indicates the day on which the conjugate was administered. The curve -●-shows the results wherein PBS was administered, and the curves -○-, -△- and -□- show the results wherein PBS containing 2 $\mu$g, 10 $\mu$g and 40 $\mu$g of the conjugate are administered, respectively. The marks "*", "**" and "***" indicate the level of significance (P) of 0.05 or less, 0.01 or less and 0.001 or less, respectively.

BEST MODE FOR CARRYING OUT THE INVENTION

The monoclonal antibody to be employed in the present invention, which specifically binds to tumor cells, is one which is rapidly internalized into the cells. That is, not less than 50%, preferably not less than 60%, still more preferably not less than 80% of the monoclonal antibody is internalized into the tumor cells within two hours after bound to the cell surfaces. Further, the monoclonal antibody must be able to make the cytotoxic component bound to the antibody exert effectively its toxicity. The monoclonal antibody may preferably be a monoclonal antibody corresponding to a glycoprotein existing on the surfaces of the human hepatocarcinoma cells, and especially preferred are AF20 and XF8 monoclonal antibodies. AF20 and XF8 are monoclonal antibodies corresponding to the surface antigen of human hepatocarcinoma cell line FOCUS [H. Takahashi et al., Hepatology 9, 625 (1989)]. It has been confirmed that these monoclonal antibodies specifically bind not only to human hepatocarcinoma cells, but also to human colon adenocarcinoma cells, human lung squamous carcinoma cells, human pancreas cancer cells and the like.

The structure of the antibody is not restricted as long as the antibody has the tumor cell-binding site, and may be, for example, IgG, F(ab')2, Fab' or Fab. The antibody may be a chimeric antibody prepared by genetic recombination technique, in which the amino acid sequence of the constant region is replaced with human amino acid sequence.

The cytotoxic component to be employed in the present invention is not restricted. Examples of the cytotoxic component include A chains of plant toxins (ribosome-inactivating protein type II) such as ricin, abrin, modeccin and viscumin; ribosome-inactivating protein type I such as saporin, luffin, momordin and Gelonin, which consist of A chains alone; A chains of bacterial proteinous toxins such as cholera toxin, heat-labile E. coli enterotoxin, pertussis toxin, tetanus toxin, botulinum toxin, Pseudomonas toxin, Shigella toxin and diphtheria toxin.

In the present invention, A chains of plant toxins such as ricin and abrin are preferably employed. Ricin is a toxin contained in castor-oil plant beans, which is one of the many proteinous toxins which exhibit strong toxicity to cells in a small amount. The ricin toxin consists of two glycoproteins, ricin A chain and ricin B chain, which are covalently bonded via disulfide bond. The ricin A chain (MW30,000) exhibits its toxicity by irreversibly stopping the protein synthesis, and the ricin B chain (MW32,000) acts as a lectin which binds to galactose-containing glycoproteins or glycolipids exposed on the cell surfaces. Since the ricin A chain contains sugar chain moiety such as mannose, the ricin A chain may contribute to the unexpected accumulation of the toxin in the liver when a conjugate of ricin and the above-mentioned monoclonal antibody is administered to a mammal. Therefore, by employing a ricin A chain in which the sugar chain is modified by an appropriate oxidizer or an enzyme, or a ricin A chain produced by E. coli or the like by the genetic recombination technique, which does not have the sugar chain, for the preparation of the immunotoxin conjugate, the half life of the conjugate in the blood may be prolonged and the accumulation of the conjugate in the liver may be prevented, so that it is preferred.

Abrin is a plant toxin contained in Abrus precatorius seeds, and similar to ricin, it exhibits strong toxicity. Abrin is categorized as ribosome-inactivating protein type II. Abrin consists of two proteins, abrin A chain and abrin B chain, which are covalently bonded via disulfide bond. Similar to ricin, the abrin A chain (MW30,000) exhibits its toxicity by irreversibly stopping the protein synthesis, and the abrin B chain (MW36,000) acts as a lectin which binds to galactose-containing glycoproteins or glycolipids exposed on the cell surfaces. However, unlike the ricin A chain, the abrin A chain does not have sugar chains at all and has an isoelectric point of 4.6 which is considerably lower than that of the ricin A chain (7.5). Thus, the abrin A chain is suited for the preparation of immunotoxin conjugates having lower isoelectric points. Such immunotoxin conjugates have an advantage in that the accumulation of the conjugate in the liver due to the existence of the sugar chain and to the high isoelectric point may be avoided.

It is preferred that the A chain to be used in the present invention be not contaminated with the B chain. The method for preparing the A chain of the toxin with such a high purity is not restricted. Examples of such method include a method in which A chain alone is produced by E. coli by employing the genetic recombination technique [M. Piatak, et al., Patent Cooperation Treaty, WO85-03508 (1986)]; a method in which the toxin is reduced so as to detach the A chain from the B chain, and purifying the A chain by using an anti-B chain antibody (Japanese Laid-open Patent Application (Kokai) No. 62-221700); and a method in which after binding the toxin to acid-treated Sepharose column, the toxin A chain is eluted with a buffer containing a reductant, and then the eluted fraction is passed through a column in which an anti-B chain antibody is fixed, so as to purify the toxin A chain (Japanese Laid-open Patent Application (Kokai) No. 62-221700). More preferred method for preparing the toxin A chain includes a method in which the toxin is adsorbed to a gel permeation chromatography column to which lactose or asialofetuin is fixed, the toxin is separated into the A chain and the B chain by using a reductant on the column, and then the A chain is eluted. After this purification of the A chain by using the lactose column or asialofetuin column, the residual B chain which may be contaminated in the purified A chain may be completely eliminated by passing the A chain fraction through the asialofetuin column or the lactose column so as to adsorb the trace amount of the toxin B chain to the second column. Thus, it is more effective to successively employ different ligand-bound gels.

It is important that the antibody in the immunotoxin conjugate of the present invention have the property to be internalized into the cells immediately after binding to the cells. By virtue of this property, the toxin B chain is not necessary and sufficient cytotoxic activity is exhibited by the toxin A chain alone. The toxin A chains such as the ricin A chain are unique RNases (RNA N-glycosidases) which inactivate ribosomes that take part in protein synthesis which is important to maintain the lives of the cells. Those substances other than the above-mentioned toxins, which inhibit reactions important for maintaining the lives of cells may also be effective for the cancer therapy. For example, special DNases, RNases and enzyme inhibitors which inhibit important intracellular enzymes may be employed.

The conjugate of the present invention may be prepared by cross-linking the toxin component and the antibody by using a cross-linking agent [Proteins, Nucleic Acids and Enzymes, extra edition, No. 31 335-343 (1987)]. As the cross-linking agent, hetero bifunctional compounds are preferred so as to assure that each of the prepared conjugates contain the both active components. Examples of such hetero bifunctional compounds include N-succinimidyl-3-(2-pyridyldithio) propionate (SPDP), m-maleimidebenzoyl-N-hydroxy-succinimidyl ester, bromoacetyl-p-aminobenzoyl-N-hydroxy-succinimidyl ester and iodoacetyl-N-hydroxy-succinimidyl ester.

The immunotoxin conjugate consisting of the toxin A chain and the antibody according to the present invention is preferred to have a property that the toxin is quickly detached from the antibody and is transferred to the site at which it exerts its activity. Thus, it is preferred to employ a cross-linking agent by which disulfide bonds are formed.

By using the immunotoxin conjugate according to the present invention, antibody-ricin A chain conjugate is introduced to the tumor cells in the body, and thereafter, the antibody is rapidly internalized into the tumor cells. Thus, the sufficient anti-tumor activity is exhibited without the ricin B chain, and the reactivity of the ricin B chain to the normal cells need not be cared at all.

Examples

The present invention will now be described in more concretely by way of examples.

Example 1

A. Antibody

As the antibodies specifically binding to the tumor cells, monoclonal antibodies XF8 and AF20 corresponding to the surface antigen of the human hepatocarcinoma cell line FOCUS, which were prepared according to the method described in H. Takahashi et al., Hepatology 9, 625 (1989) were employed.

The behaviors of the two monoclonal antibodies, especially the uptake of the monoclonal antibodies by the cells after binding of these monoclonal antibodies to the human lung squamous carcinoma cells were checked by labelling the antibodies with $^{125}$I. The labelled antibodies were prepared by the Iodogen method employing 1,3,4,6-tetrachloro-3$\alpha$-6$\alpha$-diphenylglycoluril. QG-56 cells and each of the labelled antibodies were incubated at 4°C for 3 hours to bind them. After washing the cells at 4°C, the cells were incubated at 37°C. During the incubation, the amounts of the label existing in the culture supernatant, on the cell surfaces and inside the cells were determined with time so as to trace the behaviors of the labelled antibodies. The amounts of the label in the cell supernatant were determined by counting the radioactivity of the supernatant after the reaction. The amounts of the label on the cell surfaces were determined by counting the radioactivity of the acid-papain solution with which the cells were washed, and the amounts of the label inside the cells were determined by counting the radioactivity in a buffer containing deoxycholic acid. The results are shown in Fig. 1. As can be seen from Fig. 1, either of the monoclonal antibodies was internalized into the cells within 60 - 120 minutes after binding to the cell surfaces, so that the amounts of the labelled antibodies attached to the cell surfaces were rapidly decreased.

B. Ricin A Chain

Castor-oil plant bean lectin RCA-60 purchased from Seikagaku Kogyo Co., Ltd. was fixed to a lactose-agarose column and the column was treated with PBS containing 5% mercaptoethanol (ME-PBS) overnight. Then the ricin A chain alone was eluted by ME-PBS. The eluted solution was passed through an asialofetuin column to obtain purified ricin A chain. The analysis by SDS-PAGE employing silver staining revealed that no ricin B chain was contaminated in the purified ricin A chain.

C. Preparation of Immunotoxin Conjugates

Conjugates of the ricin A chain and each of the two antibodies, that is, XF8-RA and AF20-RA were prepared by using SPDP as a cross-linking agent. More particularly, antibody solution in PBS was mixed with SPDP solution in dimethylformamide with a mixing ratio of 4:1 in terms of the molar ratio of SPDP to the antibody, and the resulting mixture was left to stand at room temperature for 30 minutes to allow the reaction, followed by gel permeation chromatography. Thereafter, three molar of ricin A chain was added into the antibody under condition of neutral pH, and the resultant was left to stand at room temperature overnight so as to allow the reaction, thereby preparing a conjugate. The obtained conjugate was purified by "Blue Sepharose CL-6B" column (commercially available from Pharmacia) and "Ultrogel AcA44" column (commercially available from IBF Biotechnics) to finally obtain purified immunotoxin conjugate. Each of the thus obtained immunotoxin conjugates contained one or two ricin A chains per one molecule of the antibody. The analysis by SDS-PAGE employing silver staining revealed that neither of the immunotoxin conjugates contained free ricin A chain.

1) Cancer Cell Growth Inhibition Effects by Antibody-Ricin A Chain Conjugates

Various cancer cell lines were cultured under the normal conditions. In a 96-well plate, cancer cells were placed in the population density of 3 x $10^4$ cells/well, and the conjugates were placed in the wells at various concentrations, followed by overnight incubation. The activities of the conjugates were determined by measuring the inhibition of the uptake of $^3$H-leucine by the cells, which is contained in the culture medium. As a result, as shown in Fig. 2 A (QG-56, human lung squamous carcinoma cells) and B (LS-180, human colon adenocarcinoma cells), especially AF20-RA in the two kinds of antibody-ricin A chain conjugate exhibited strong activity almost equal to that of complete ricin (A chain + B chain) in spite of the lack of the ricin B chain. Thus, it was shown that the antibody effectively contributes to the internalization of the ricin A chain into the cells.

2) Anti-tumor Effects of Antibody-Ricin A Chain Conjugates for Cancer-Bearing Nude Mice

Small clusters of human lung squamous carcinoma cells were transplanted to Balb/c nude mice of 6 weeks old. Fifteen days after, each of the two kinds of the antibody-ricin A chain conjugate was administered to the mice 4 times at doses of 2, 10 or 40 μg/mouse, and the changes in the tumor weight and body weight were measured. On the 31st day from the transplantation, the mice were sacrificed. The real weight of the tumors was measured and the outer appearances of the livers were observed. However, no abnormal signs were observed. As shown in Fig. 3, the changes in the tumor weight very well correspond to the cancer cell growth inhibition effects (the results shown in Fig. 2 A). Thus, AF20-RA exhibited higher anti-tumor effect than XF8-RA.

Example 2

A. Antibody

As the monoclonal antibody specifically binding to the tumor cells, monoclonal antibody AF20 was used.

B. Abrin A Chain

Pulverized Abrus precatorius seeds were defatted by the extraction with petroleum ether, and then immediately extracted with water. In the extraction, the pH was adjusted to 4.5 with acetic acid. After centrifugation, the supernatant was recovered and was successively subjected to precipitation with ammonium sulfate (40 - 70%), to affinity chromatography by acid-treated Sepharose 4B, and to that by DEAE cellulose, so as to purify abrin. The thus obtained abrin was reduced with Tris buffer (pH 7.7) containing 5% mercaptoethanol at room temperature for 3 hours to overnight. Thereafter, the pH was adjusted to 8.5 and the resultant was passed through DEAE cellulose column, followed by extensive washing. Thereafter, elution was performed with buffer (pH 8.5) containing 0 - 0.2M NaCl, and the eluted solution was then passed through asialofetuin column, followed by gel permeation chromatography, thereby obtaining purified abrin A chain. The analysis by SDS-PAGE employing silver staining revealed that no abrin B chain was contaminated in the purified abrin A chain.

C. Preparation of Immunotoxin Conjugate

Conjugate of the abrin A chain and AF20 antibody, that is, AF20-AA was prepared by using SPDP as a cross-linking agent. More particularly, antibody solution in PBS was mixed with SPDP solution in dimethylformamide with a mixing ratio of 4:1 in terms of the molar ratio of SPDP to the antibody, and the resulting mixture was left to stand at room temperature for 30 minutes to allow the reaction, followed by gel permeation chromatography. Thereafter, three molar of abrin A chain was added into the antibody, and the resultant was left to stand at room temperature overnight so as to allow the reaction, thereby preparing a conjugate. The obtained conjugate was purified by "Blue Sepharose CL-6B" column and then by "Ultrogel AcA44" column as in Example 1 to finally obtain purified immunotoxin conjugate. The thus obtained immunotoxin conjugate contained one or two abrin A chains per one molecule of the antibody. The analysis by SDS-PAGE employing silver staining revealed that the immunotoxin conjugate did not contain free ricin A chain.

1) Cancer Cell Growth Inhibition Effects by Antibody-Abrin A Chain Conjugate

QG-56 cells (human squamous carcinoma cells) were cultured under the normal conditions. In a 96-well plate, QG-56 cells were placed in the population density of $3 \times 10^4$ cells/well, and the conjugate was placed in the wells at various concentrations, followed by overnight incubation. The activities of the conjugates were determined by measuring the inhibition of the uptake of $^3$H-leucine by the cells, which is contained in the culture medium. As a result, as shown in Fig. 4, AF20-AA exhibited activity 3.5 times higher than that exhibited by AF20-ricin A chain conjugate (AF20-RA).

2) Anti-tumor Effects of Antibody-Ricin A Chain Conjugates for Cancer-Bearing Nude Mice

Small clusters of human lung squamous carcinoma cells were transplanted to Balb/c nude mice of 6 weeks old. Fifteen days after, AF20-AA was administered to the mice 4 times at doses of 2, 10 or 40 $\mu$g/mouse, and the changes in the tumor weight and body weight were measured. On the 31st day from the transplantation, the mice were sacrificed. The real weight of the tumors was measured and the outer appearances of the livers and other major organs were observed. The results are shown in Fig. 5.

INDUSTRIAL APPLICABILITY

The immunotoxin conjugate of the present invention can specifically send a cytotoxic component to the tumor cells and can make the tumor cells take the conjugate into the cells so as to effectively kill the tumor cells. Thus, the immunotoxin conjugate of the present invention is useful as a pharmaceutical for treating cancers.

**Claims**

1. An immunotoxin conjugate comprising an antibody which binds to a tumor cell and which has a property to be easily internalized into the tumor cell, and A chain of a toxin bound to the antibody.

2. The immunotoxin conjugate of claim 1, wherein said antibody is a monoclonal antibody directed to human hepatocarcinoma cell.

3. The immunotoxin conjugate of claim 2, wherein said human hepatocarcinoma cell is FOCUS.

4. The immunotoxin conjugate of claim 2, wherein said antibody is AF20 or XF8.

5. The immunotoxin conjugate of claim 1, wherein said antibody has a property that not less than 50% of the antibody is internalized into said tumor cell within 2 hours after binding to said tumor cell.

6. The immunotoxin conjugate of claim 1, wherein the A chain of said toxin is A chain of ricin or abrin.

7. The immunotoxin conjugate of claim 1, wherein said antibody and the A chain of said toxin are bonded by using a cross-linking agent.

8. The immunotoxin conjugate of claim 1, wherein said tumor cell is human colon cancer cell, human lung cancer cell, human hepatocarcinoma cell or human pancreas cancer cell.

Fig. 1

I C$_{50}$ (M)

| | | | |
|---|---|---|---|
| Ricin | : $1.3 \times 10^{-11}$ | XF8-RA | : $1.6 \times 10^{-9}$ |
| RA (Ricin A Chain) | : $2.2 \times 10^{-7}$ | AF20-RA | : $5.2 \times 10^{-11}$ |

Fig. 2 (A)

$I\,C_{50}\ (M)$

| | | | |
|---|---|---|---|
| Ricin | : $1.5 \times 10^{-11}$ | XF8$-$RA | : $6.7 \times 10^{-10}$ |
| RA (Ricin A Chain) | : $2.1 \times 10^{-7}$ | AF20$-$RA | : $9.4 \times 10^{-12}$ |

Fig. 2 (B)

Fig. 3

Fig. 4

Fig. 5

# INTERNATIONAL SEARCH REPORT

International Application No PCT/JP91/00874

---

**I. CLASSIFICATION OF SUBJECT MATTER** (if several classification symbols apply, indicate all) [6]

According to International Patent Classification (IPC) or to both National Classification and IPC

Int. Cl$^5$  A61K37/02, A61K39/395, A61K45/00

---

**II. FIELDS SEARCHED**

Minimum Documentation Searched [7]

| Classification System [ | Classification Symbols |
|---|---|
| IPC | A61K37/02-A61K37/04, A61K39/395-A61K39/44, A61K45/00-A61K45/08 |

Documentation Searched other than Minimum Documentation
to the Extent that such Documents are Included in the Fields Searched [8]

---

**III. DOCUMENTS CONSIDERED TO BE RELEVANT** [9]

| Category* \| | Citation of Document, [11] with indication, where appropriate, of the relevant passages [12] | Relevant to Claim No. [13] |
|---|---|---|
| X | JP, A, 61-12630 (Sanofi), January 21, 1986 (21. 01. 86) & US, A, 4670563 & FR, B, 2566271 | 1, 6, 7 |
| X | JP, A, 63-115826 (Zoma CORP), May 15, 1988 (15. 05. 88) | 1, 6, 7 |
| Y | JP, A, 61-12630 (Sanofi), January 21, 1986 (21. 01. 86) | 2, 3, 8 |
| Y | JP, A, 63-115826 (Zoma CORP), May 15, 1988 (15. 05. 88) | 2, 3, 8 |
| A | JP, A, 64-43199 (Shionogi & Co., Ltd.), February 15, 1989 (15. 02. 89) | 4, 5 |

---

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance: the claimed invention cannot be considered novel or cannot be considered to involve an inventive step

"Y" document of particular relevance: the claimed invention cannot be considered to involve an inventive step when the document is combined with one or more other such documents, such combination being obvious to a person skilled in the art

"&" document member of the same patent family

---

**IV. CERTIFICATION**

| Date of the Actual Completion of the International Search | Date of Mailing of this International Search Report |
|---|---|
| September 6, 1991 (06. 09. 91) | September 24, 1991 (24. 09. 91) |

| International Searching Authority | Signature of Authorized Officer |
|---|---|
| Japanese Patent Office | |

Form PCT/ISA 210 (second sheet) (January 1985)